# EUROPÄISCHE PATENTSCHRIFT

(11) **EP 0 691 332 B1**
(45) Veröffentlichungstag und Bekanntmachung des Hinweises auf die Patenterteilung: **08.09.1999**
(21) Anmeldenummer: 95109981.1
(22) Anmeldetag: 27.06.1995
(51) Int. Cl.: C07D 231/16, C07D 231/12, A01N 43/56

(54) **Pyrazolylderivate, ihre Herstellung und ihre Verwendung als Schädlingsbekämpfungsmittel**
Pyrazolyl derivatives, their preparation and their use as pesticides
Dérivés pyrazolyl, leur préparation et leur utilisation comme pesticide

(30) Priorität: 06.07.1994 DE 4423615
(43) Veröffentlichungstag der Anmeldung: 10.01.1996
(73) Patentinhaber: BASF AKTIENGESELLSCHAFT, 67056 Ludwigshafen (DE)
(72) Erfinder: Kirstgen, Reinhard, Dr., D-67434 Neustadt (DE); König, Hartmann, Dr., D-69115 Heidelberg (DE); Sauter, Hubert, Dr., D-68167 Mannheim (DE); Harries, Volker, Dr., D-67227 Frankenthal (DE); Lorenz, Gisela, Dr., D-67434 Hambach (DE); Ammermann, Eberhard, Dr., D-64646 Heppenheim (DE)

(56) Entgegenhaltungen:
- EP-A- 0 398 692
- EP-A- 0 477 631
- DE-A- 4 238 260

## Beschreibung

Die vorliegende Erfindung betrifft Pyrazolylderivate der allgemeinen Formel I 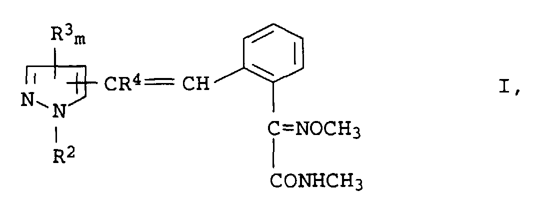 in der der Index und die Substituenten die folgende Bedeutung haben:
- m: 0, 1 oder 2, wobei die Reste R³ verschieden sein können, wenn m größer als 1 ist;
- R²: ggf. durch Halogen, Cyano, C₁-C₄-Alkyl, C₁-C₂-Halogenalkyl, C₁-C₄-Alkoxy, C₁-C₂-Halogenalkoxy, Phenyl oder Oxy-C₁-C₂-alkylidenoxy substituiertes Phenyl;
- R³: Nitro, Cyano, Halogen, C₁-C₄-Alkyl, C₁-C₄-Halogenalkyl, C₁-C₄-Alkoxy oder C₁-C₄-Halogenalkoxy;
- R⁴: Wasserstoff, Cyano, Halogen, C₁-C₄-Alkyl oder C₁-C₄-Halogenalkyl.

Außerdem betrifft die Erfindung Verfahren zur Herstellung dieser Verbindungen, sie enthaltende Mittel und Verfahren zur Bekämpfung von Schädlingen mit diesen Mitteln.

Aus der Literatur sind 2-Alkoxyiminoessigsäureamide mit fungizider Wirkung bekannt (EP-A 398 692, EP-A 468 775, WO-A 92/13,830, WO-A 93/07,116, WO-A 93/08,180, GB-A 2 253 624, JP-A 05/255,012, WO-A 94/05,626, JP-A 05/294,948). Außerdem werden in EP-A 463 488, EP-A 477 631, EP-A 567 828 und DE-A 42 38 260 derartige Derivate mit insektizider, akarizider oder nematodizider Wirkung beschrieben. Die Wirkung der bekannten Derivate ist allerdings nicht immer befriedigend.

Der vorliegenden Erfindung lagen daher Verbindungen mit verbesserter Wirksamkeit als Aufgabe zugrunde.

Demgemäß wurden die eingangs definierten Verbindungen gefunden. Außerdem wurden Verfahren zu ihrer Herstellung sowie ihre Verwendung zur Bekämpfung von Schädlingen gefunden.

Die Herstellung der Verbindungen I erfolgt in Analogie zu verschiedenen, aus der Literatur an sich bekannten Methoden. Bei der Herstellung ist es unerheblich, ob zunächst die Gruppierung (Pyrazolyl)-CR⁴=CH-(Phenyl) oder die Gruppierung -C(=NOCH₃)-CONHCH₃ aufgebaut wird. Besonders bevorzugt erhält man diese Gruppierungen nach den nachfolgend beschriebenen Verfahren, wobei zur besseren Übersichtlichkeit die jeweils nicht an der Umsetzung beteiligte Gruppe vereinfacht dargestellt ist [(Pyrazolyl)-CR⁴=CH- ≡ R*; -C(=NOCH₃)-CONHCH₃ ≡ R^{#}].

### A: Verfahren zum Aufbau der Gruppierung (Pyrazolyl)-CR⁴=CH-:

Die (Pyrazolyl)-CR⁴=CH- Gruppierung erhält man durch Umsetzung eines geeigneten Aldehyds entweder mit einer Triphenylphosphoniumverbindung im Sinne einer Wittig Reaktion oder mit einem Phosphonat im Sinne einer Wittig-Horner Reaktion.

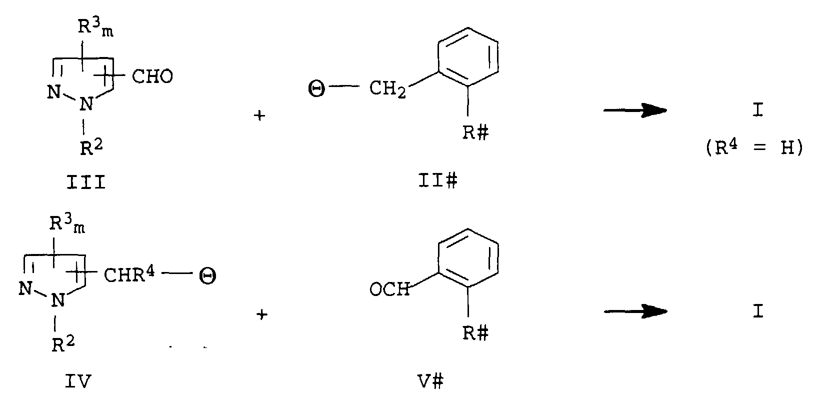

Θ in den Formeln II und IV steht für ^{⊕}P(C₆H₅)₃ ^{⊖}Hal, wobei Hal ein Halogenatom wie insbesondere Chlor, Brom oder Iod bedeutet, oder für PO(OR^{a})₂, wobei R^{a} eine C₁-C₈-Alkylgruppe, insbesondere C₁-C₄-Alkyl, bedeutet.

Die Umsetzungen werden üblicherweise bei Temperaturen von 0°C bis 80°C, vorzugsweise 20°C bis 60°C, durchgeführt.

Geeignete Lösungsmittel sind beispielsweise aromatische Kohlenwasserstoffe (z.B. Toluol, o-, m- und p-Xylol), halogenierte Kohlenwasserstoffe (z.B. Methylenchlorid, Chloroform, Tetrachlorkohlenstoff und Chlorbenzol), Ether (z.B. Diethylether, Diisopropylether, tert.-Butylmethylether, Dioxan, Anisol und Tetrahydrofuran), Nitrile (z.B. Acetonitril und Propionitril), Alkohole (z.B. Methanol, Ethanol, Propanol, Isopropanol, Butanol und tert.-Butanol), Dimethylsulfoxid, Dimethylformamid, Dimethylacetamid, 1,3-Dimethylimidazolidin-2-on und 1,3-Dimethyltetrahydro-2(1H)-pyrimidin, besonders bevorzugt Toluol, Tetrahydrofuran, Methanol und Dimethylformamid. Es können auch Gemische der genannten Lösungsmittel verwendet werden.

Als Basen kommen allgemein anorganische Verbindungen wie Alkalimetall- und Erdalkalimetallhydride (z.B. Lithiumhydrid, Natriumhydrid, Kaliumhydrid und Calziumhydrid) und Alkalimetallamide (z.B. Lithiumamid, Natriumamid und Kaliumamid) oder metallorganische Verbindungen wie Alkalimetallalkyle (z.B. Methyllithium, Butyllithium und Phenyllithium), Alkylmagnesiumhalogenide (z.B. Methylmagnesiumchlorid) sowie sowie Alkalimetall- und Erdalkalimetallalkoholate (z.B. Natriummethanolat, Natriumethanolat, Kaliumethanolat, Kalium-tert.-butanolat und Dimethoxy-magnesium), außerdem organische Basen wie tertiäre Amine (z.B. Trimethylamin, Triethylamin, Diisopropylethylamin, N-Methylpiperidin sowie bicyclische Amine) in Betracht. Besonders bevorzugt werden Natriumhydrid, Natriumamid, Natriummethanolat, Kaliummethanolat und Kalium-tert.-butanolat verwendet.

Es kann für die Umsetzung vorteilhaft sein, eine katalytische Menge eines Kronenethers (z.B. 18-Krone-6 oder 15-Krone-5) zuzusetzen.

Die Basen werden im allgemeinen äquimolar, im Überschuß oder gegebenenfalls als Lösungsmittel verwendet.

Die Edukte werden üblicherweise in äquimolaren Mengen eingesetzt. Es kann für die Vervollständigung der Umsetzung vorteilhaft sein, eines der Edukte in einem Überschuß von 1 mol-% bis 100 mol-%, vorzugsweise 1 mol-% bis 20 mol-%, bezogen auf das zweite Edukt einzusetzen.

Die Herstellung der Ausgangsverbindungen II erfolgt ausgehend von den entsprechenden Benzylchloriden bzw. -bromiden oder -iodiden durch Umsetzung mit Triphenylphosphin oder Trialkylphosphit gemäß literaturbekannten Verfahren (vgl. Houben-Weyl, Methoden der organischen Chemie Bd. 12/1, Seite 579 ff bzw. Seite 433 ff, Verlag Thieme, Stuttgart 1963).

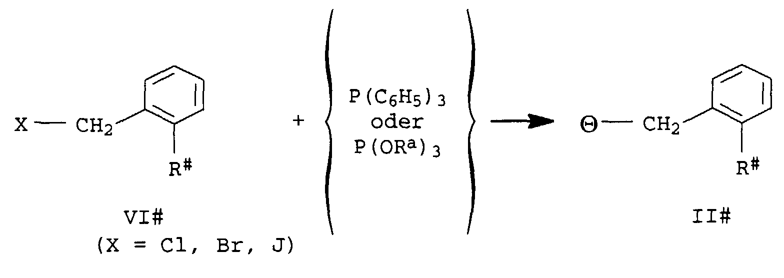

Die Herstellung der Benzylverbindungen ist in EP-A 477 631 beschrieben.

Die Pyrazolderivate III bzw. IV sind aus der Literatur bekannt oder können nach den dort beschriebenen Verfahren hergestellt werden [vgl. J. Heterocycl. Chem. 24, 739 (1987); J. Heterocycl. Chem. 25, 555 (1988); J. Heterocycl. Chem. 28, 1545 (1991); Bull. Chem. Soc. Jpn. 58, 1841 (1985); Bull. Chem. Soc. Jpn. 59, 2631 (1986); Chem. Lett. 1982, 543; J. Chem. Soc. 1957, 3314; Liebigs Ann. Chem. 1985, 1377; Synthesis 1983, 566].

Die jeweils benötigte funktionelle Gruppe (Aldehyd bzw. Methylenphosphorverbindung) kann durch Formylierung [vgl. Pharmazie 30, 157 (1975)], Reduktion und Oxidation [vgl. Heterocycles 23, 1417 (1985); J. Het. Chem. 27, 1933 (1990)], Halogenierung [vgl. J. Het. Chem. 14, 1171 (1977)] und Umsetzung mit Triphenylphosphin oder Trialkylphosphit erhalten werden.

Verbindungen IV, in denen R⁴ Halogen bedeutet, können analog den in EP-A 544 587 beschriebenen Methoden erhalten werden.

### B: Verfahren zum Aufbau der Gruppierung -C(=NOCH₃)-CONHCH₃:

Man erhält die Verbindungen I durch Aminolyse der entsprechenden 2-Methoxyimino-phenylessigsäureester VII (vgl. Houben-Weyl Bd. E5, Seite 983 ff).

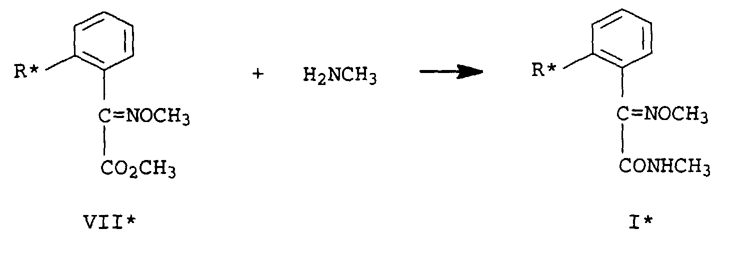

Die Umsetzung wird üblicherweise bei Temperaturen von 0°C bis 60°C, vorzugsweise 10°C bis 30°C, durchgeführt.

Methylamin kann entweder als Gas eingeleitet oder als wäßrige Lösung einer Lösung der Verbindungen VII zudosiert werden.

Geeignete Lösungsmittel sind aromatische Kohlenwasserstoffe (z.B. Toluol, o-, m- und p-Xylol), halogenierte Kohlenwasserstoffe (z.B. Methylenchlorid, Chloroform, Tetrachlorkohlenstoff und Chlorbenzol), Ether (z.B. Diethylether, Diisopropylether, tert.-Butylmethylether, Dioxan, Anisol und Tetrahydrofuran) und Alkohole (z.B. Methanol, Ethanol, Propanol, Isopropanol, Butanol und tert.-Butanol), besonders bevorzugt Methanol, Toluol und Tetrahydrofuran. Es können auch Gemische der genannten Lösungsmittel verwendet werden.

Weitere Verfahren zur Herstellung dieser Gruppierung sind aus der eingangs zitierten Literatur bekannt.

Die Umwandlung in die Gruppierung -C(=NOCH₃)-CONHCH₃ kann auf jeder der mit # gekennzeichneten Zwischen- oder Vorstufen erfolgen.

Die Verbindungen I können bei der Herstellung aufgrund ihrer C=C- und C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen eine Trennung nicht erforderlich, da sich die einzelnen Isomere während der Aufbereitung für die Anwendung oder bei der Anwendung (z.B. durch Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schädling erfolgen.

In Bezug auf die C=N-Doppelbindung werden hinsichtlich ihrer Wirksamkeit die E-Isomeren der Verbindungen bevorzugt (Konfiguration bezogen auf die OCH₃-Gruppe im Verhältnis zur CONHCH₃-Gruppe).

In Bezug auf die CR⁴=CH-Doppelbindung werden hinsichtlich ihrer Wirksamkeit die "E"-Isomere bevorzugt (Konfiguration bezogen auf den Pyrazolrest im Verhältnis zum Phenylring).

Bei der eingangs angegebenen Definition der Verbindungen I wurden Sammelbegriffe verwendet, die allgemein repräsentativ für die folgenden Gruppen stehen:
Halogen: Fluor, Chlor, Brom und Jod;
Alkyl: gesättigte, geradkettige oder verzweigte Kohlenwasserstoffreste mit 1 bis 4 oder 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 4 Kohlenstoffatomen (wie vorstehend genannt), welche über ein Sauerstoffatom (-O-) an das Gerüst gebunden sind;
Halogenalkoxy: geradkettige oder verzweigte Halogenalkylgruppen mit 1 bis 4 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind;
Alkenyl: ungesättigte, geradkettige oder verzweigte Kohlenwasserstoffreste mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl: geradkettige oder verzweigte Kohlenwasserstoffgruppen mit 2 bis 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung werden Verbindungen I bevorzugt, in denen m für 0 oder 1 steht.

Die vorliegende Erfindung betrifft Verbindungen I, in denen R² für ggf. subst. Phenyl steht. Als Substituenten des Phenylrests kommen die folgenden Gruppen in Betracht: Halogen, Cyano, C₁-C₄-Alkyl, C₁-C₂-Halogenalkyl, C₁-C₄-Alkoxy, C₁-C₂-Halogenalkoxy, Phenyl oder Oxy-C₁-C₂-alkylidenoxy.

Des weiteren werden Verbindungen I bevorzugt, in denen R³ für Cyano, Halogen, C₁-C₂-Alkyl, C₁-C₂-Halogenalkyl, C₁-C₂-Alkoxy oder C₁-C₂-Halogenalkoxy steht.

Insbesondere werden Verbindungen I bevorzugt, in denen R³ für Chlor, Methyl oder Trifluormethyl steht.

Gleichermaßen werden Verbindungen I bevorzugt, in denen R⁴ für Wasserstoff oder Halogen steht.

Besonders werden Verbindungen I bevorzugt, in denen R⁴ für Wasserstoff, Fluor oder Chlor steht.

Von besonderer Bedeutung sind auch Verbindungen I, in denen die Gruppierung (Pyrazolyl)-CR⁴=CH- für eine der folgenden Gruppen W.1 bis W.23 steht:

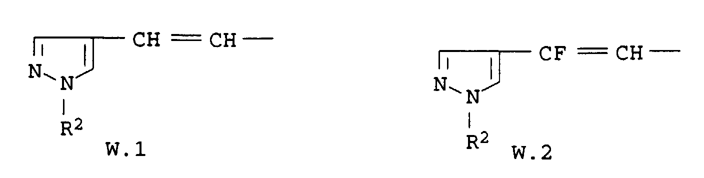

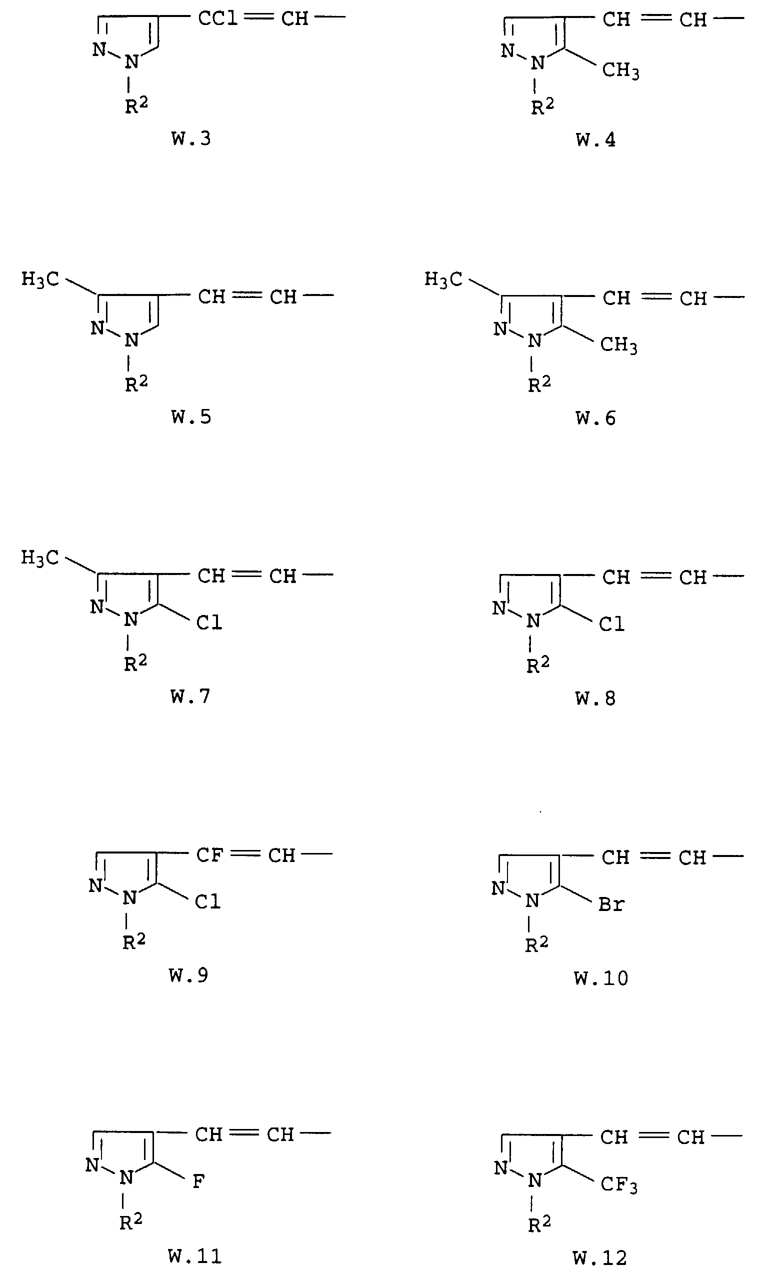

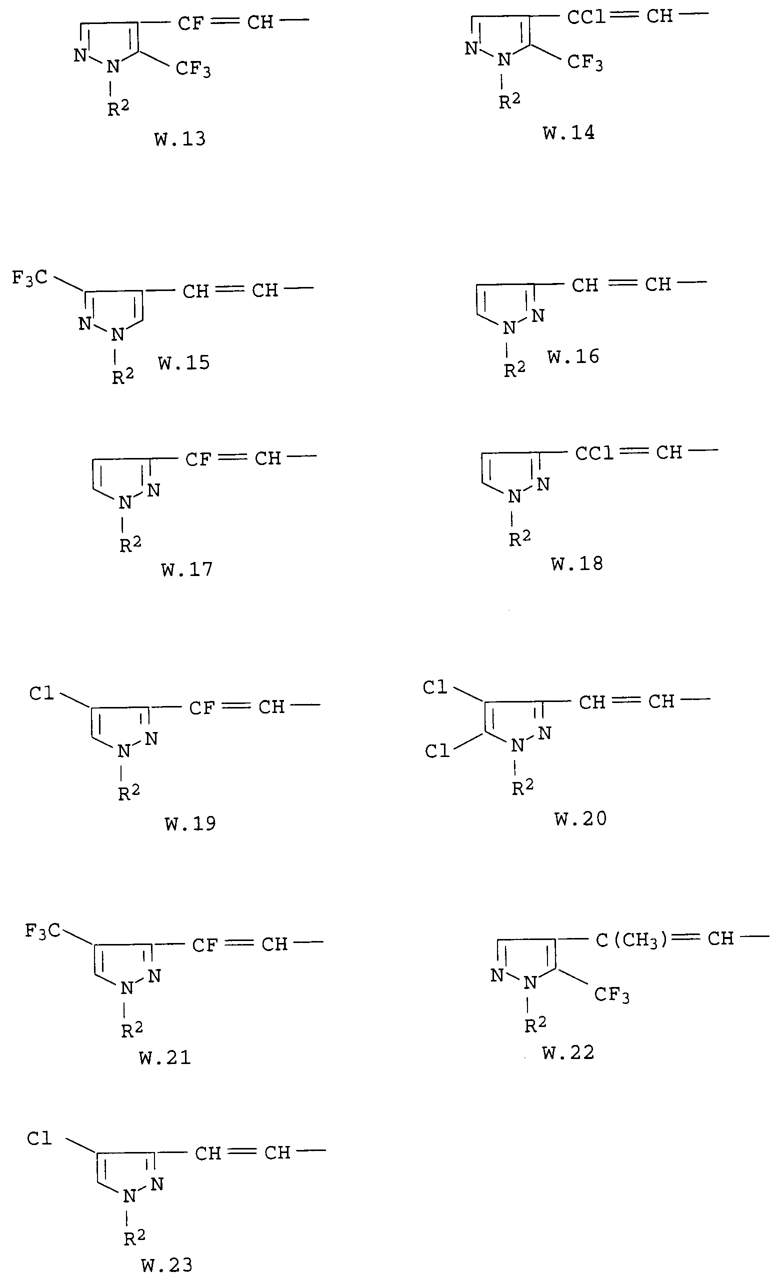

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumifecana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiginella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliacia, Hyphantria cunea, Hyponomeuta malinellus, Keiferia lycopecsicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pestinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Octiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granacia.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lincilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissins leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificins, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Disperstonen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg um Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritspulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

### Beispiele für Formulierungen sind:

I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele:

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1

### E-2-Methoxyimino-2-[(2-methylphenyloxymethyl)-phenyl]-essigsäuremethylamid

250 g E-2-Methoxyimino-2-[(2-methylphenyloxymethyl)-phenyl]-essigsäuremethylester werden in 1 l 40 %ige wäßrige Methylaminlösung suspendiert und 4 h auf 40°C erwärmt. Nach Abkühlen auf Raumtemperatur (20°C) wird der Feststoff abgesaugt, mehrmals mit Wasser nachgewaschen und bei 50°C getrocknet. Man erhält 229,8 g der Titelverbindung als farblose Kristalle.
Fp.: 109 - 112°C
¹H-NMR (CDCl₃, δ in ppm):
   2,20 (s, 3H); 2,85 (d, 3H); 3,95 (s, 3H); 4,90 (s, 2H); 6,7 (NH); 6,8 - 7,6 (m, 8H)

### Beispiel 2

### E-2-Methoxyimino-2-[(2-chlormethyl)-phenyl]-essigsäuremethylamid

2 g E-2-Methoxyimino-2-[(2-methylphenyloxymethyl)-phenyl]-essigsäuremethylamid aus Beispiel 1 werden in 30 ml wasserfreiem Dichlormethan bei 10°C vorgelegt. Hierzu tropft man 9,4 g Bortrichloridlösung (1molare Lösung in n-Hexan), erhitzt 1,5 h auf Rückfluß, kühlt auf 10°C ab, gibt nochmals 9,4 g Bortrichloridlösung zu und läßt über Nacht bei Raumtemperatur (20°C) rühren. Nach Zutropfen von 8,2 g Methanol wird der Ansatz einrotiert. Der Rückstand wird in 100ml Dichlormethan aufgenommen, mit 5 % Natronlauge und dann mit Wasser gewaschen. Die organ. Phase wird abschließend über Natriumsulfat getrocknet. Nach Abziehen des Lösungsmittels verbleiben 1,2 g der Titelverbindung als Öl.
¹H-NMR (CDCl₃, δ in ppm):
   2,95 (d, 3H); 3,90 (s, 3H); 4,45 (s, 2H); 6,8 (NH); 7,1 - 7,6 (m, 4H)

### Beispiel 3

### E-2-Methoxyimino-2-[(2-brommethyl)-phenyl]-essigsäuremethylamid

10 g E-2-Methoxyimino-2-[(2-methylphenyloxymethyl)-phenyl]-essigsäuremethylamid aus Beispiel 1 werden in 50 ml wasserfreiem Dichlormethan vorgelegt. In die Lösung wird Bromwasserstoff bis zur Sättigungskonzentration (ca. 9 g HBr) eingeleitet. Nach 68 h Rühren bei Raumtemperatur ist das Edukt vollständig umgesetzt. Nach Zugabe von weiteren 50 ml Dichlormethan wird wie in Beispiel 2 aufgearbeitet. Es verbleiben 7,0 g der Titelverbindung als Öl, welches beim Stehenlassen durchkristallisiert.
Fp.: 128 - 129°C
¹H-NMR (CDCl₃, δ in ppm):
   2,95 (d, 3H); 3,95 (s, 3H); 4,35 (s, 2H); 6,85 (NH); 7,1 - 7,5 (m, 4H)

### Beispiel 4

### E-2-Methoxyimino-2-[(2-dimethylphosphonomethyl)-phenyl]-essigsäuremethylamid

15 g E-2-Methoxyimino-2-[(2-brommethyl)-phenyl]-essigsäuremethylamid werden in 50 ml Trimethylphosphit 3 Stunden auf 110°C erhitzt. Nach vollständigem Umsatz des Bromids wird überschüssiges Trimethylphosphit bei ca. 15 Torr abdestilliert. Der Rückstand wird in 200 ml Dichlormethan aufgenommen, mehrmals mit gesättigter NaCl-Lösung gewaschen und über Natriumsulfat getrocknet. Nach Abziehen des Lösungsmittels verbleiben 12,8 g eines zähen, braunen Öls.
¹H-NMR (CDCl₃, δ in ppm):
   2,9 (d, 3H); 3,0 (s, 1H); 3,15 (s, 1H); 3,55 (s, 3H); 3,65 (s, 3H); 3,95 (s, 3H); 7,0-7,5 (m, 5H)

### Beispiel 5

### E-2-Methoxyimino-2-[2-(E-2-[1-(4-chlorphenyl)-pyrazol-4-yl]-ethenyl)-phenyl]-essigsäuremethylamid

3,1 g 1-(4-Chlorphenyl)-4-formylpyrazol und 5,65 g des Phosphonats aus Beispiel 4 wurden in 30 ml wasserfreiem Dimethylformamid gelöst. Hierzu gibt man bei Raumtemperatur 0,43 Natriumhydrid. Der Ansatz schäumt auf, wobei sich die Temperatur von 21°C auf 38°C erhöht. Nach weiteren 2 Stunden Rühren wird der Ansatz auf Eiswasser gegossen. Der dabei ausfallende Niederschlag wird abgesaugt und in Essigsäureethylester aufgenommen. Die organische Phase wird mehrmals mit gesättigter Natriumchlorid-Lösung gewaschen, über Na₂SO₄ getrocknet und einrotiert. Der zurückbleibende Feststoff wird mit 40 ml Essigsäureethylester verrührt und abgesaugt. Man erhält 3,0 g der Titelverbindung als farblosen Feststoff.
Fp.: 188 - 190°C
¹H-NMR (CDCl₃, δ in ppm):
   2,95 (d, 3H); 4,0 (s, 3H); 6,7 (d, 1H); 6,8 (br, NH); 6,9 (d, 1H); 7,15 (d, 1H); 7,25-7,7 (m, 7H); 7,8 (s, 1H); 7,9 (s, 1H)

### Beispiel 6

### E-2-Methoxyimino-2-[2-(E-2-[1-(2,4-dichlorphenyl)-5-methyl-pyrazol-4-yl]-ethenyl)-phenyl]-essigsäuremethylamid

3,8 g 1-(2,4-Dichlorphenyl)-4-formyl-5-methyl-pyrazol und 4,7 g des Phosphonats aus Beispiel 4 werden in 80 ml wasserfreiem Dimethylformamid analog Beispiel 5 umgesetzt. Man erhält 3,4 g Rohprodukt, wobei nach Verreiben mit Diisopropylether 2,2 g der Titelverbindung als farbloser Feststoff verbleiben.
Fp.: 165 - 168°C
¹H-NMR (CDCl₃, δ in ppm):
   2,2 (s, 3H); 2,95 (d, 3H); 4,0 (s, 3H); 6,7 (d, 1H); 6,8 (br, NH); 6,9 (d, 1H); 7,2 (d, 1H); 7,3-7,5 (m, 4H); 7,6 (s, 1H); 7,7 (d, 1H); 7,85 (s, 1H)

### Beispiel 7

### E-2-Methoxyimino-2-[2-(E-2-[1-(2,4-dichlorphenyl)-pyrazol-3-yl]-ethenyl)-phenyl]-essigsäuremethylamid

3,6 g 1-(2,4-Dichlorphenyl)-3-formyl-pyrazol und 4,7 g des Phosphonats aus Beispiel 4 werden in 80 ml wasserfreiem Dimethylformamid analog Beispiel 5 umgesetzt. Man erhält 4,2 g Rohprodukt, aus dem das E-Isomer säulenchromatographisch (Kieselgel, Laufmittel Toluol/Essigsäureethylester 9:1 auf 8:2, v/v) isoliert werden kann.
E-Isomer: 2,0 g farbloser Feststoff
Fp.: 151 - 152°C
¹H-NMR (CDCl₃, δ in ppm):
   2,9 (d, 3H); 3,95 (s, 3H); 6,6 (d, 1H); 6,7 (br, NH); 6,9 (d, 1H); 7,0-7,6 (m, 8H); 7,75 (d, 1H); 7,9 (d, 1H)

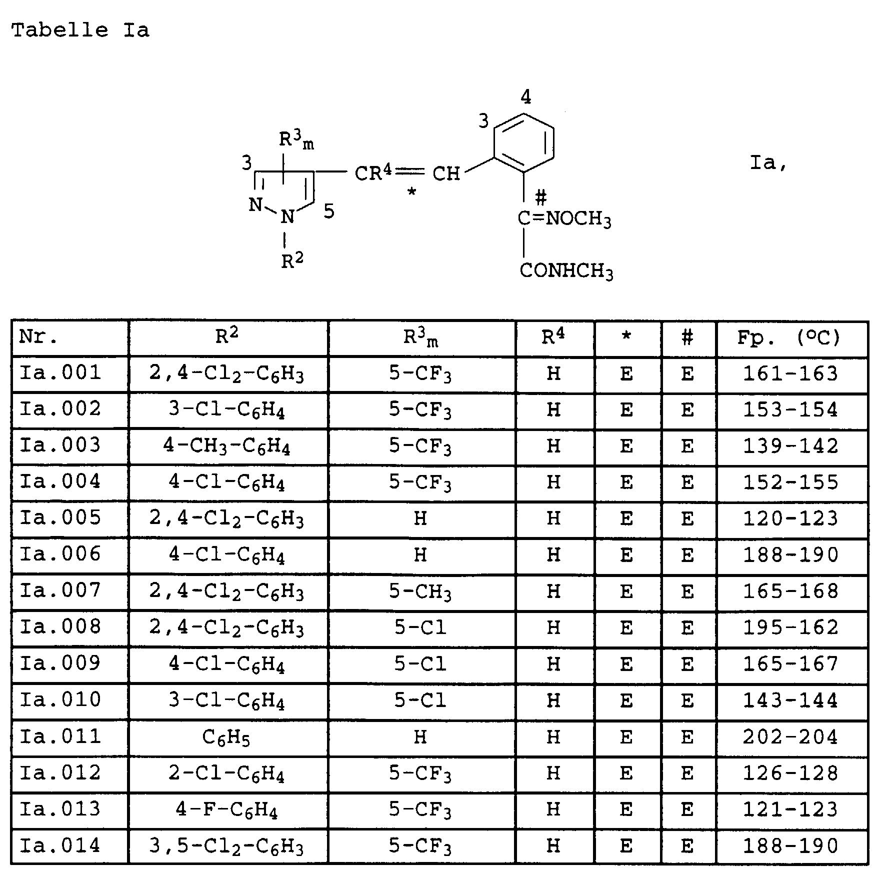

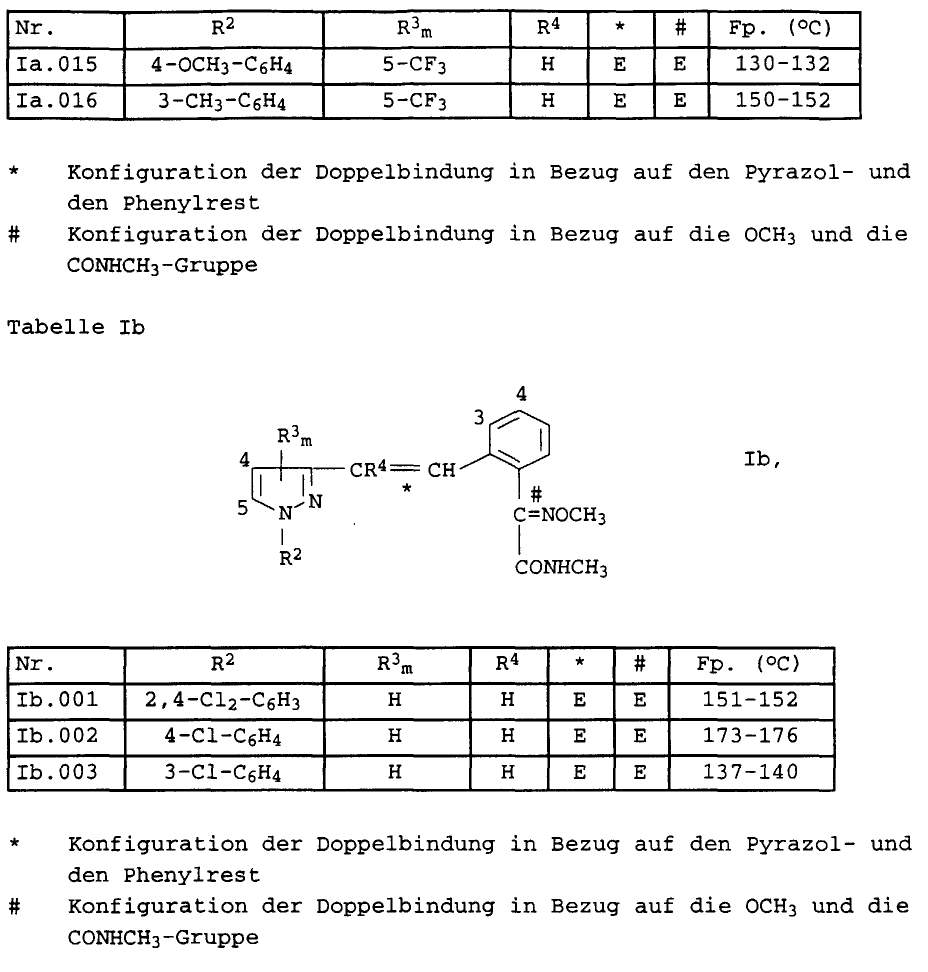

### Anwendungsbeispiele

Die insektizide Wirkung der Verbindungen der allgemeinen Formel I ließ sich durch folgende Versuche zeigen:

### Die Wirkstoffe wurden

a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

### Wirkung gegen Pyricularia oryzae (Reisbrand)

Reiskeimlinge (Sorte: "Tai Nong 67") wurden mit der Wirkstoffaufbereitung (Aufwandmenge: 250 ppm) tropfnaß gespritzt. Nach 24 Stunden wurden die Pflanzen mit einer wäßrigen Sporensuspension des Pilzes *Pyricularia oryzae* besprüht und 6 Tage bei 22-24 °C bei einer relativen Luftfeuchtigkeit von 95-99 % bewahrt. Die Beurteilung erfolgte visuell.

In diesem Test zeigten die mit den Verbindungen Ia.007, Ia.008, Ia.010, Ia.011, Ia.012 und Ia.013 behandelten Pflanzen einen Befall von 15% und weniger während die unbehandelten Pflanzen zu 70% befallen waren.

### Wirkung gegen Plasmopara viticola (Rebenperonospora)

Topfreben (Sorte: "Müller Thurgau") wurden mit der Wirkstoffaufbereitung (Aufwandmenge: 63 ppm) tropfnaß gespritzt. Nach 8 Tagen wurden die Pflanzen mit einer Zoosporenaufschwemmung des Pilzes *Plasmopara viticola* besprüht und 5 Tage bei 20-30 °C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Test zeigten die mit den Verbindungen Ia.008, Ia.009, Ia.010, Ia.011, Ia.012, Ia.013, Ia.015 und Ia.016 behandelten Pflanzen einen Befall von 5% und weniger während die unbehandelten Pflanzen zu 80% befallen waren.

## Patentansprüche

1. Pyrazolylderivate der allgemeinen Formel I 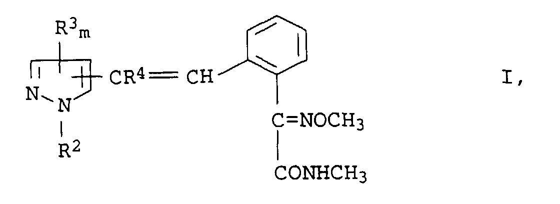 in der der Index und die Substituenten die folgende Bedeutung haben:
m 0, 1 oder 2, wobei die Reste R³ verschieden sein können, wenn m größer als 1 ist;
R² ggf. durch Halogen, Cyano, C₁-C₄-Alkyl, C₁-C₂-Halogenalkyl, C₁-C₄-Alkoxy, C₁-C₂-Halogenalkoxy, Phenyl oder Oxy-C₁-C₂-alkylidenoxy, substituiertes Phenyl;
R³ Nitro, Cyano, Halogen, C₁-C₄-Alkyl, C₁-C₄-Halogenalkyl, C₁-C₄-Alkoxy oder C₁-C₄-Halogenalkoxy;
R⁴ Wasserstoff, Cyano, Halogen, C₁-C₄-Alkyl oder C₁-C₄-Halogenalkyl.

2. Verfahren zur Herstellung der Verbindungen I gemäß Anspruch 1, in denen R⁴ Wasserstoff bedeutet, dadurch gekennzeichnet, daß man ein Phosphorderivat der allgemeinen Formel II 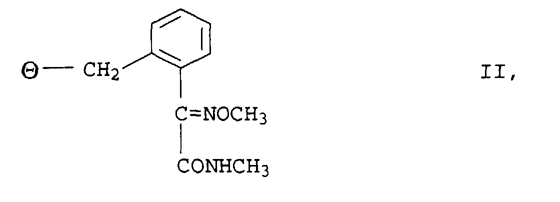 in der Θ für ^{⊕}P(C₆H₅)₃ ^{⊖}Hal (Hal = Halogen) oder PO(OR^{a})₂ (R^{a} = C₁-C₈-Alkyl) steht, in einem inerten organischen Lösungsmittel in Gegenwart einer Base mit einem Aldehyd der allgemeinen Formel III umsetzt.

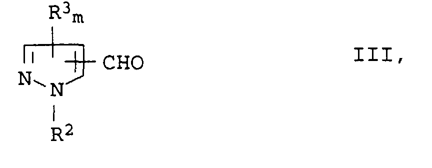

3. Verfahren zur Herstellung der Verbindungen I gemäß Anspruch 1, dadurch gekennzeichnet, daß man ein Pyrazolderivat der Formel IV 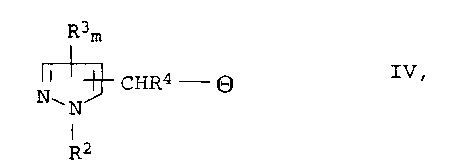 in der Θ die in Anspruch 2 gegebene Bedeutung hat, in einem inerten organischen Lösungsmittel in Gegenwart einer Base mit einem Benzaldehyd der Formel V 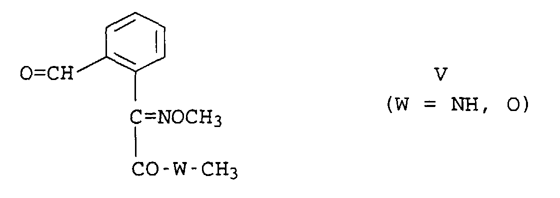 umsetzt und für den Fall, daß W Sauerstoff bedeutet, anschließend den erhaltenen Oximetherester der Formel VII 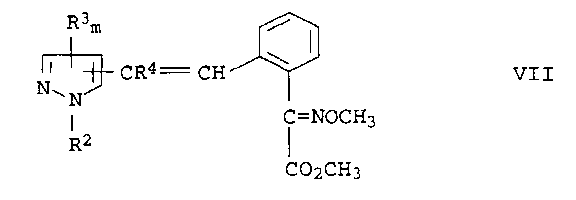 mit Methylamin oder einem Methylammoniumsalz in I überführt.

4. Zur Bekämpfung von Schädlingen geeignetes Mittel, enthaltend einen festen oder flüssigen Trägerstoff und eine Verbindung der allgemeinen Formel I gemäß Anspruch 1.

5. Verwendung der Verbindungen I gemäß Anspruch 1 zur Herstellung eines zur Bekämpfung von Schädlingen geeigneten Mittels.

6. Verfahren zur Bekämpfung von Schädlingen, dadurch gekennzeichnet, daß man die Schädlingen oder die von ihnen zu schützenden Materialien, Pflanzen, Boden oder Saatgüter mit einer wirksamen Menge einer Verbindung der allgemeinen Formel I gemäß Anspruch 1 behandelt.

7. Verwendung der Verbindungen I gemäß Anspruch 1 zur Bekämpfung von Schädlingen.

## Claims

1. A pyrazolyl derivative of the general formula I 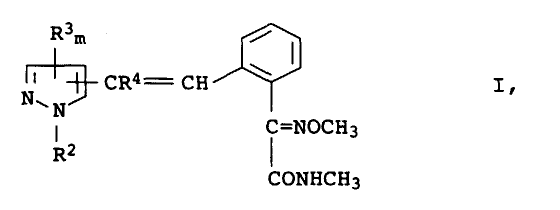 where the index and the substituents have the following meanings:
m is 0, 1 or 2, it being possible for the radicals R³ to be different if m is greater than 1;
R² is phenyl which is unsubstituted or substituted by halogen, cyano, C₁-C₄-alkyl, C₁-C₂-haloalkyl, C₁-C₄-alkoxy, C₁-C₂-haloalkoxy, phenyl or oxy-C₁-C₂-alkylidenoxy;
R³ is nitro, cyano, halogen, C₁-C₄-alkyl, C₁-C₄-haloalkyl, C₁-C₄-alkoxy or C₁-C₄-haloalkoxy;
R⁴ is hydrogen, cyano, halogen, C₁-C₄-alkyl or C₁-C₄-haloalkyl.

2. A process for preparing the compounds I as claimed in claim 1, where R⁴ is hydrogen, which comprises reacting a phosphorus derivative of the general formula II 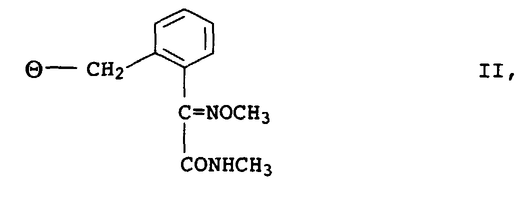 where Θ is ^{⊕}P(C₆H₅)₃ ^{⊖}Hal (Hal = halogen) or PO(OR^{a})₂ (R^{a} = C₁-C₈-alkyl) with an aldehyde of the general formula III 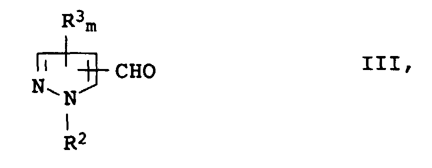 in an inert organic solvent in the presence of a base.

3. A process for preparing the compounds I as claimed in claim 1, which comprises reacting a pyrazole derivative of the formula IV 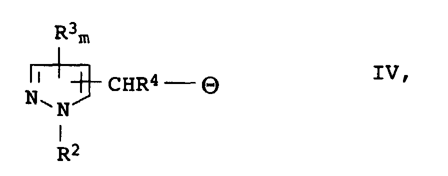 where Θ has the meaning given in claim 2, with a benzaldehyde of the formula V 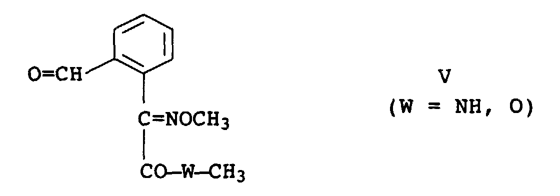 in an inert organic solvent in the presence of a base and in the case where W is oxygen, then converting the resulting oxime ether ester of the formula VII 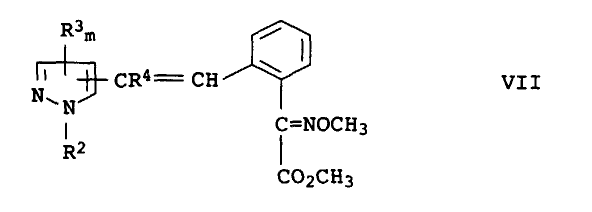 to I using methylamine or a methylammonium salt.

4. A composition suitable for controlling pests, containing a solid or liquid carrier and a compound of the general formula I as claimed in claim 1.

5. The use of the compounds I as claimed in claim 1 for preparing a composition suitable for controlling pests.

6. A method of controlling pests, which comprises treating the pests or the materials, plants, soil or seed to be protected from them with an active amount of a compound of the general formula I as claimed in claim 1.

7. The use of the compounds I as claimed in claim 1 for controlling pests.

## Revendications

1. Dérivés pyrazolyliques de formule générale I 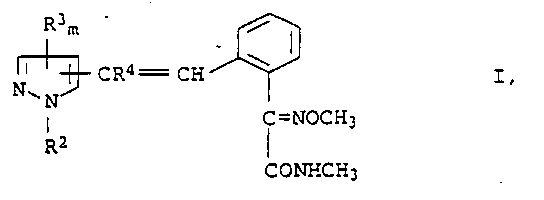 dans laquelle l'indice et les symboles ont les significations suivantes :
m : 0, 1 ou 2, les symboles R³ pouvant avoir des significations différentes lorsque m est supérieur à 1 ;
R² : phényle portant éventuellement des substituants halogéno, cyano, alkyle en C1-C4, halogénoalkyle en C1-C2, alcoxy en C1-C4, halogénoalcoxy en C1-C2, phényle ou oxy-(alkylidène en C1-C2)-oxy ;
R³ : nitro, cyano, halogéno, alkyle en C1-C4, halogénoalkyle en C1-C4, alcoxy en C1-C4 ou halogénoalcoxy en C1-C4 ;
R⁴ : hydrogène, cyano, halogène, alkyle en C1-C4 ou halogénoalkyle en C1-C4.

2. Procédé de préparation des composés I selon la revendication 1 pour lesquels R⁴ représente l'hydrogène, caractérisé par le fait que l'on fait réagir un dérivé du phosphore de formule générale II 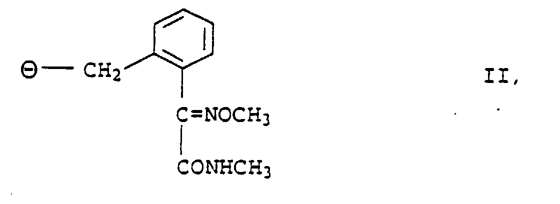 dans laquelle Θ représente ^{⊕}P(C₆H₅)₃ ^{⊖}Hal (Hal = halogène) ou PO(OR^{a})₂ (R^{a} = alkyle en C1-C8) dans un solvant organique inerte et en présence d'une base, avec un aldéhyde de formule générale III

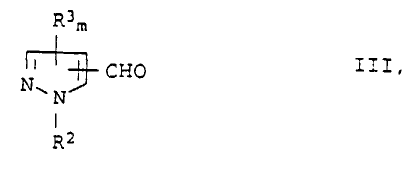

3. Procédé de préparation des composés I de la revendication 1, caractérisé par le fait que l'on fait réagir un dérivé du pyrazole répondant à la formule IV 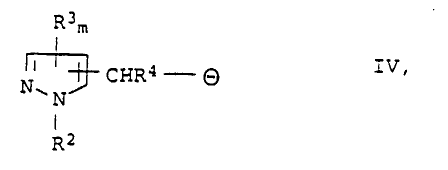 dans laquelle Θ a les significations indiquées dans la revendication 2, dans un solvant organique inerte et en présence d'une base, avec un benzaldéhyde de formule V 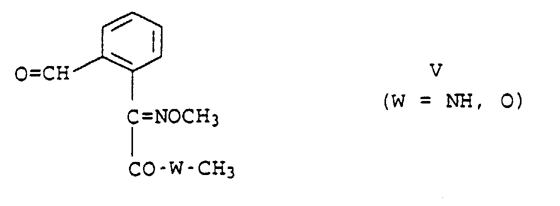 et, lorsque W représente l'oxygène, on convertit ensuite l'éther-ester d'oxime obtenu, répondant à la formule VII 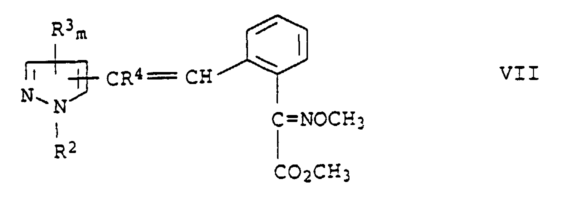 en le composé I à l'aide de la méthylamine ou d'un sel de méthylammonium.

4. Produit convenant à l'utilisation pour la lutte contre les parasites, contenant un véhicule solide ou liquide et un composé de formule générale I de la revendication 1.

5. Utilisation des composés I de la revendication 1 pour la préparation d'un produit convenant à l'utilisation pour la lutte contre les parasites.

6. Procédé pour combattre les parasites, caractérisé par le fait que l'on traite les parasites ou les matériaux, végétaux, sols ou semences qu'on veut protéger contre les parasites par une quantité efficace d'un composé de formule générale I de la revendication 1.

7. Utilisation des composés I de la revendication 1 pour la lutte contre les parasites.
